# EUROPEAN PATENT SPECIFICATION

(11) **EP 2 303 192 B1**
(45) Date of publication and mention of the grant of the patent: **14.11.2018**
(21) Application number: 09731923.0
(22) Date of filing: 03.04.2009
(51) Int. Cl.: A61F 2/30, A61F 2/38

(54) **METHOD FOR MANUFACTURING AN IMPLANT**
METHODE ZUR HERSTELLUNG EINES IMPLANTATS
PROCÉDÉ POUR FABRIQUER UN IMPLANT

(30) Priority: 16.04.2008 US 103824
(43) Date of publication of application: 06.04.2011
(73) Proprietor: Biomet Manufacturing, LLC, Warsaw, IN 46582 (US)
(72) Inventor: SCHOENEFELD, Ryan, J., Fort Wayne, IN 46804 (US); SALYER, Brian, D., Warsaw, IN 46580 (US); CANADA, Michael, Akron, IN 46910 (US); KIPHART, Rory, Warsaw, IN 46582 (US); METZGER, Robert, Wakarusa, IN 46573 (US); WHITE, John, R., Winona Lake, IN 46590 (US)
(74) Representative: Mays, Julie
(86) International application number: PCT/US2009/039507
(87) International publication number: WO 2009/129063

(56) References cited:
- WO-A-91/07139
- WO-A-2005/051240
- WO-A-2008/044055
- WO-A-2008/101090
- WO-A1-01/84479
- WO-A2-03/051210
- DE-A1- 4 421 153
- US-A- 4 695 283

## Description

### INTRODUCTION

Various methods of manufacturing patient specific and off-the shelf implant components are known. WO01/84479 discloses a method and system for scanning a surface and generating a three-dimensional object. WO03/051210 discloses a system and method for joint resurface repair.

The present teachings provide a surgeon-interactive manufacturing method that includes various implant options.

### SUMMARY

The invention is defined in claim 1. Preferred embodiments are shown in the dependent claims. The present invention relates to an orthopedic implant manufacturing method. The method includes preparing a preliminary pre-operative surgical plan for a specific patient, communicating the plan to a surgeon of the patient, and receiving an orthopedic implant design recommendation of the surgeon.

The preliminary pre-operative surgical plan for a specific patient includes selecting one of a first, second and third option, the first option being a patient-specific implant customised for the specific patient, the second option being a semi-custom implant that has at least one patient-independent feature and at least one patient-specific feature, and the third option being an off-the-shelf implant available only in predetermined sizes.

The method further includes sending a request for manufacturing the selected implant to a manufacturing center, manufacturing the selected implant based on the request. receiving the implant, and forwarding the implant for implantation.

Also described is an orthopedic implant manufacturing method which includes providing a generic casting of a specific implant component, the generic casting having at least one geometric feature that can be machined to a plurality of different sizes of the implant component, the generic casting including size-independent features of the specific component, and machining the component to a patient-specified size.

Also described is a device that includes a generic casting for a specific implant component, the generic casting being intermediate between stock material and a specific size implant component. The generic casting includes at least one size-independent feature of the implant component, and at least one feature machinable to size/shape for a specific patient.

Further areas of applicability of the present teachings will become apparent from the description provided hereinafter. It should be understood that the description and specific examples are intended for purposes of illustration only and are not intended to limit the scope of the present teachings.

### BRIEF DESCRIPTION OF THE DRAWINGS

The present teachings will become more fully understood from the detailed description and the accompanying drawings, wherein:
FIG. 1 is a flowchart of an implant manufacturing method according to the present invention;
FIG. 2 is a diagram illustrating a computer interface for an implant manufacturing method according to the present invention;
FIG. 3 is perspective view of a generic casting of an implant;
FIG. 4 is a side view of a generic casting; and
FIG. 5 is a plan view of a generic casting.

### DESCRIPTION

The following description is merely exemplary in nature and is in no way intended to limit the present teachings, applications, or uses. For example, although some of the present teachings are illustrated for a knee implant, the present teachings can be used for any orthopedic implant.

The present teachings provide a manufacturing method that integrates patient's anatomic and medical information with interactive participation by a surgeon to select and manufacture an implant and, optionally, related surgical instruments, for a particular patient from generally three options: a custom made implant specific to the patient; an implant that is only partially custom-made or a semi-custom implant, and a standard off-the self implant. Similarly, off-the-self or custom-made or semi-custom made instrumentation, such as alignment guides, drill guides, cutting guides or other instruments can be selected and manufactured, as recommended by the surgeon, for the surgical procedure. All the implant components, alignment guides and other disposable instruments can be included in a package provided to a surgeon for a specific patient.

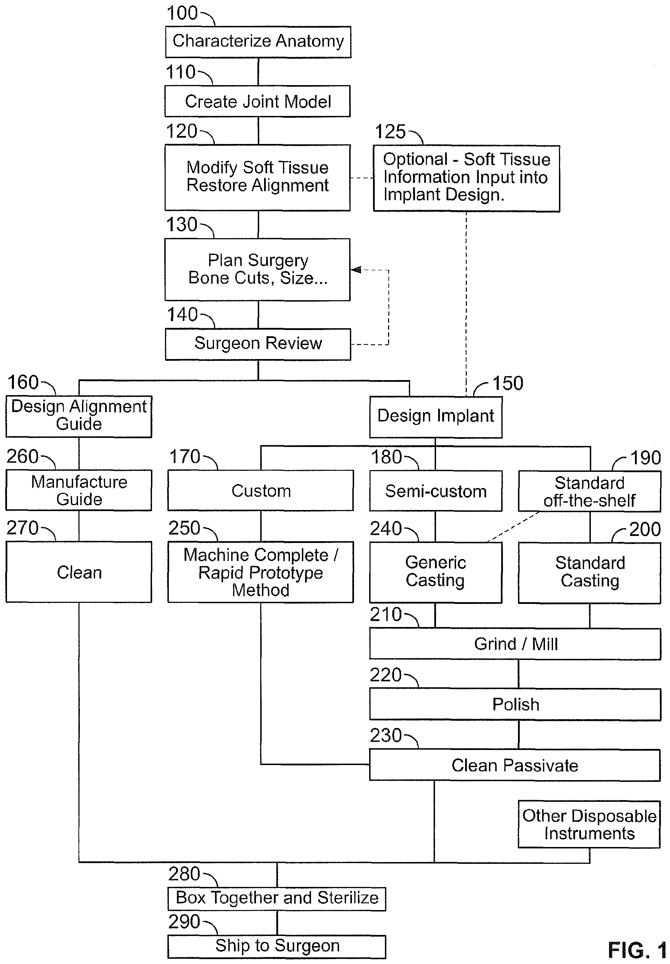

Referring to FIG. 1, an exemplary flowchart of an interactive implant manufacturing method according to the present teachings is illustrated. At 100, the portion of the patient's anatomy related to the orthopedic procedure and the implant is characterized and detailed with various imaging methods capable of obtaining a representation of the affected anatomy, including, for example, soft and hard tissues, such as bone, or bone joints with or without cartilage, ligaments or other soft tissue. The imaging methods can include, for example, MRI, CT, ultrasound, radiography or X-ray, cameras and other devices. The image information for the patient can be obtained at a medical facility or a doctor's office and can be sent to the manufacturer in an electronic/digital form contained in a memory storage medium, such as a CD, DVD, memory stick, CF or SD card or other storage device, or as an electronic file transmitted over the Internet or worldwide web or by using any other electronic communication methods, including e-mail or other digital transmission to any time of computer device, smart phone, PDA or other devices in which electronic information can be transmitted.

With continued reference to FIG. 1, at 110, the information collected at 100 can be used to create a three-dimensional model or image of the bone or joint with or without associated soft tissue or related anatomy using commercially available computer modeling software from various vendors or developers, such as, for example, from Materialise USA, Ann Arbor, Michigan. The three-dimensional model of the patient's anatomy can be viewed on a computer display or other electronic screen and can also reproduced as a hard copy on film or other medium and viewed by direct or indirect or backlight illumination.

At 120, soft tissue associated with the affected anatomy can be modified, or removed or repaired, to restore alignment of the joint, for example, or to remove torn or diseased tissue, or to cut or repair ligaments, or to provide natural or artificial ligament grafts. Soft tissue information can be optionally used as an additional design parameter or input for the implant design, at 125. For example, a custom or patient-specific bearing articulation of a knee joint can be designed based on the kinematic profile and the soft tissue/ligament information available for a particular patient. Further, kinematic information for the patient can be obtained by an actual gait analysis of the patient, and can also be obtained by computer modeling software that uses the MRI images of the patient's joints and associated ligaments, muscle or other soft tissue to derive kinematic analysis of the patient and corresponding recommendations for soft tissue modification, such as releasing a ligament, for example. Such software is commercially available from the Biomechanics Research Group, Inc., of San Clemente, CA.

At 130, a preliminary pre-operative plan of the surgical procedure can be prepared, including the planning of various bone resections, sizes and types of implants, and various geometric requirements including relevant dimensions, such as height, width, orientation of particular features, etc. The preliminary pre-operative surgical plan can include a recommendation of particular implants and associated instruments to be used in the surgical procedure, as discussed below. The preliminary pre-operative surgical plan can be in the form of digital images that can be viewed interactively using a computer modeling software, such as the software referenced above.

At 140, the preliminary pre-operative surgical plan can be submitted to the surgeon for review, either electronically or by land mail, and either in digital or hard copy form, as discussed above in connection with transmitting imaging information. Based on the preliminary pre-operative surgical plan and the patient information, the surgeon can make a recommendation regarding the design of the implant at 150, and any desired associated alignment guides at 160. At 150, the surgeon can recommend a method of designing an implant. Specifically, the surgeon can select one of the following three options: a first option of a custom or patient-specific implant at 170, or a second option of a semi-custom made implant at 180, or a third option of a standard or off-the-shelf implant at 190. It will be appreciated that, based on the surgeon's recommendation at 140, the preliminary pre-operative surgical plan can be modified at 130 and then resubmitted to the surgeon for approval.

A custom-made implant is a patient-specific, one of a kind implant specifically made for a particular patient, and consequently there is no inventory associated with such implant. Standard or off-the-self-implants are available and stocked in a number of sizes, typically six or more, and a number of configurations or types, including bilateral or unilateral implants, constrained, semi-constrained, mobile, etc. Because of the variety of sizes and configurations that are kept in stock to be accommodate different patients, a large inventory of standard implants is created, and several molds for each type and size of implant may be used. As described below in detail, semi-custom implants provide an intermediate solution between custom-made and off-the-self implants. Semi-custom implants reduce the size of inventory and molds required for production, while allowing some degree of patient-specific customization.

Custom or patient-specific implants, when approved by surgeon at 170 for a specific patient, can be manufactured for the patient by rapid prototyping methods, such as stereolithography or other similar methods, or by CNC milling, or other automated or computer-controlled machining, or by robotic methods, at 250. Manufacturing can take place at a manufacturing center or facility in situ or at remote or off-site location. It will be understood that in situ manufacturing is used as a short hand for a manufacturing site of the original equipment manufacturer (OEM), but can be physically located at a different facility of the OEM. Off-site or remote manufacturing will be understood to refer to facilities operated by other manufacturers who are contracted by the OEM for manufacturing all or some of the components or parts for the surgical procedure.

Off-the-self implants, when approved by the surgeon at 190, can be manufactured by standard casting methods from bar stock or other stock material at 200, then shaped to a final shape and size by grinding or milling at 210, polished at 220, and then cleaned/passivated at 230. Such off-the-self implants can be part of an existing inventory, or mass-produced, or produced by just-in-time agile manufacturing methods.

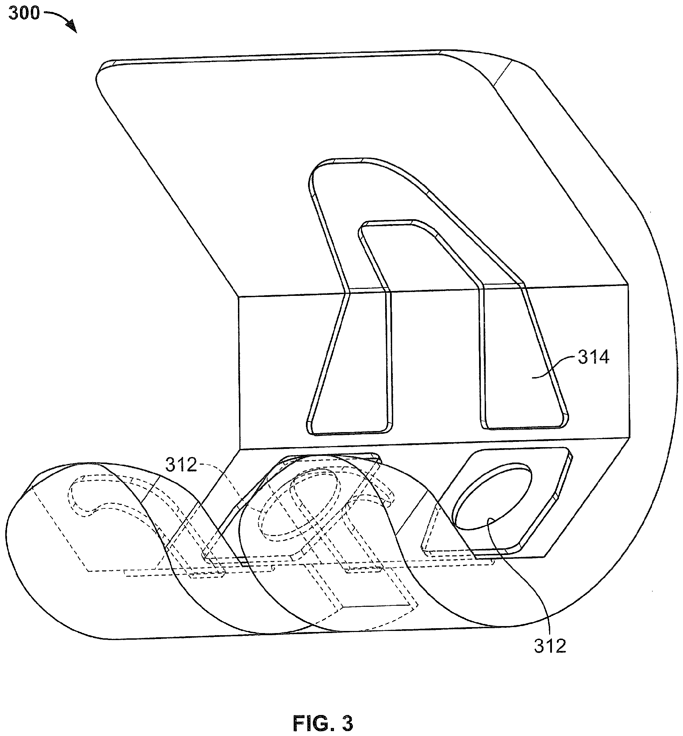

Semi-custom implants, when approved by the surgeon at 180, can be made from a generic casting at 240, as described below, or by modifying existing standard implant designs to match various features or parameters based on the anatomy of the patient, as described in co-pending patent application entitled Patient-Modified Implant and Associated Methods, Serial No. 12/103,834, filed on April 16, 2008 (Published as US 2008/0262624). After the generic casting is modified for certain parameters of a patient, it can be processed at aspects 210-230 to a passivated form. Patient- specific parameters can include parameters relating to the size of the implant, including height, width, various articulation parameters or angles, etc., as discussed in specific example below in reference to FIGS. 3-5.

The surgeon's review of the surgical plan at 140 may further include, at 160, a request for one or more patient-specific alignment guides to be used with the implant. Patient-specific alignment guides are described in co- pending patent applications Serial No. 11/756057, filed on May 31, 2007 (Published as US 2007/0288030), Serial No. 11/971390, filed on January 9, 2008 (Published as US 2008/0312659), No. 12/025414, filed on February 4, 2008 (Published as US 2008/0114370), and Serial No. 12/039849 filed on February 29, 2008 (Published as US 2008/0161815). The alignment guides can be manufactured at 260 with by rapid prototyping methods, such as stereolithography or other similar methods or by CNC milling, or other automated
or computer-controlled machining or robotic methods, and cleaned at 270. The alignment guides, the implants and optionally other disposable instruments can be packaged and sterilized at 280, and forwarded to the surgeon or the surgeon's medical facility for implantation at 290.

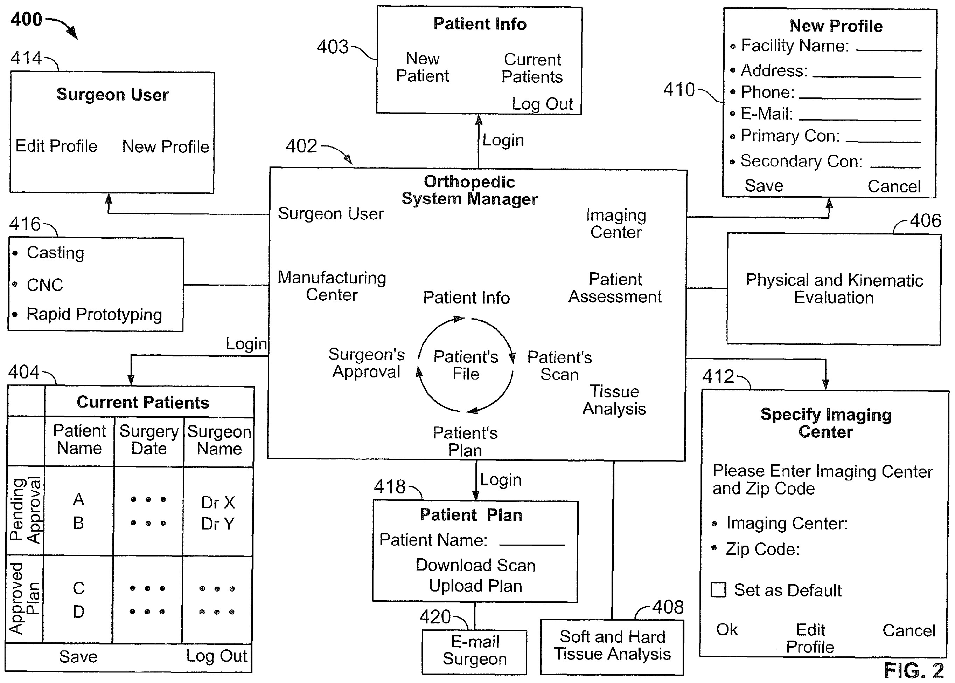

Referring to FIG. 2, a computer interface 400 to a computer program for the management of the manufacturing method is illustrated diagrammatically. An orthopedic system manager 402 can be in the form of software or other computer program associated with the original equipment manufacturer. The orthopedic system manager 402 can be accessible locally via dedicated computer machines or computer terminal directly communicated with software either by hard wire or wirelessly. The orthopedic system manager 402 can also be accessible remote remotely via the Internet or other remote communication portals using any electronic or other devices that can connect to the Internet or other web-based network, or other similar communication networks, including cable, satellite and telephone-based networks.

The system manager 402 can provide access to patient file information, including lists of all current patients at 403, and surgery dates, surgeons, and approval status of the surgical plan for each patient, at 404. Each patient file can include personal and medical information of the patient, such as, for example, weight, height, gender, age, lifestyle, pertinent medical records and medical history, as well as information on patient assessment that includes physical and kinematic evaluation pertaining to the orthopedic procedure at 406, and soft and hard tissue analysis at 408, including information provided at aspects 120 and 125 of FIG. 1, as discussed above. Imaging center information for patient scans, as discussed in relation to aspects 100 and 110 of FIG. 1, can added or modified at 410, and an imaging center for each specific patient can be specified at 412. Surgeon profiles, including surgeon preferences regarding anatomic axes alignment or implant and instrument preferences that can be taken into account when preparing the preliminary pre-operative plan discussed at aspect 130 of FIG. 1, can be created and edited at 414. Information and selection of manufacturing centers can be accessed at 416 for manufacturing the implants and or alignment guides as discussed in relation to aspects 260, 250, 240, and 210-230 of FIG. 1. The preliminary pre-operative surgical plan for each patient can be provided at 418, as discussed above at 140 in reference to FIG. 1, and e-mailed or otherwise communicated to the patient's surgeon at 420.

As discussed above at aspects 150 to 190 of FIG. 1, one implant option includes manufacturing semi-custom implants by generic casting. Illustrative examples of generic casting of a semi-custom femoral component are shown in FIGS. 3-5. A generic casting 300 of the implant is a casting that is more specialized than ordinary bar stock, from which any size of component can be made, but less specialized than the off-the-self components that are available in a particular number of sizes, typically six-to ten sizes and are finished from specific castings of those sizes. The generic casting can be made in a size and shape that can accommodate a range of variable features for the component, and at the same time can be machined to multiple sizes, such as three or four smaller sizes. In contrast, off-the-self implants require a mold or casting for each offered size, and a larger inventory of available sizes for each implant component. The generic casting can generally include geometric features which are size/shape and/or patient-independent or universal, and also features that are size/shape or patient-specific, as discussed in the examples below. More particularly, the generic casting can include at least one geometric feature that will remain unchanged for any patient or universal feature, and at least one geometric feature that can be specifically customized for and is specific to a particular patient.

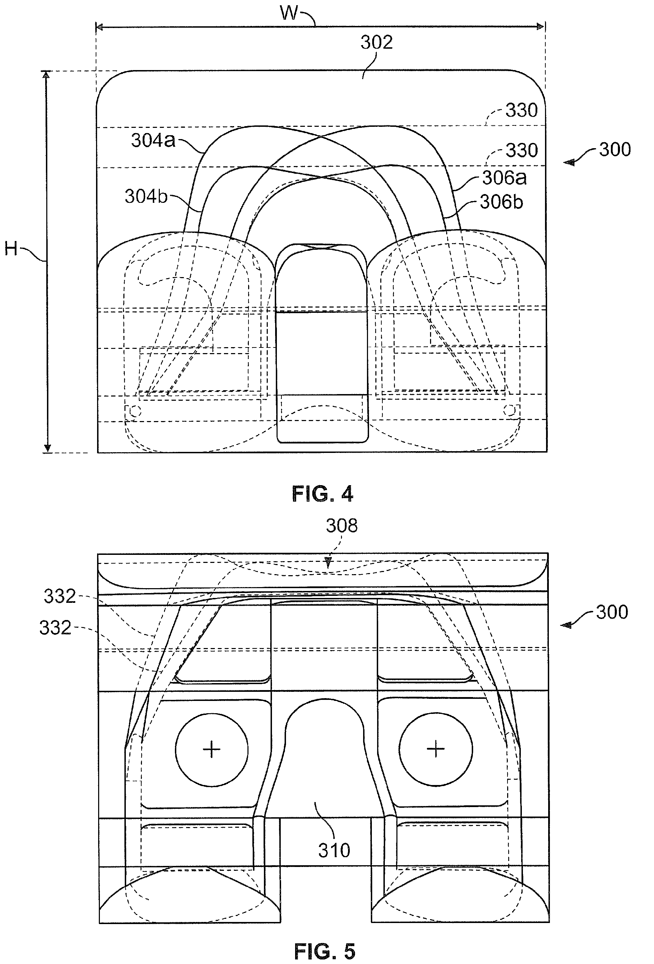

Referring to FIGS. 4 and 5, an exemplary generic casting 300 of a femoral component is illustrated. In this example, the generic casting 300 can have an anterior flange 302 of medial-lateral width W, and/or a height H and/or other geometric dimensions to accommodate multiple sizes of femoral components. For example, multiple sizes of left-sided implants 304a, 304b, and various sizes of right-sided implants 306a, 306b can be formed by a single generic casting. Appropriate markings or indentations or score lines for cutting to size can be provided, such as height markings 330, for example. The implant for a particular patient can be formed from the generic casting 300 by selecting particular features, such as the width W or height H, or other geometric features for a particular patient and machining the generic casting 300 to provide the size, dimension or shape, or combinations thereof for that particular geometric feature.

Referring to FIG. 5, the generic casting 300 does not include a patella track feature, but provides an area in which a custom patella track 308 can be machined at a custom angle for each specific patient. The generic casting 300 can also include additional material in the inner condylar notch area 310 to allow for custom machining of the intercondylar notch area 310 to accommodate various types of articulation or constraint in relation to a tibial component, such cams or intercondylar boxes, and other contact areas for articulation with the tibial component in accordance with a kinematic plan for the joint of the specific patient. Separate molds for posterior stabilized and cruciate retaining articulations can be made, each mold capable of accommodating multiple sizes of the corresponding implant type. For example, the intercondylar notch area 310 can be machined for line or area contact with the articular surfaces of a tibial component of various degrees of flexion. Exemplary articulations are disclosed in commonly assigned U.S. Patents No. 6,589,283, No. 6,413,279, and No. 6,165,223, and in co-pending U.S. Patent Application Ser. No. 10/840,765 filed on May 6, 2004. Various markings 332 corresponding to different sizes can be provided.

Referring to FIG. 3, the generic casting 300 can include at least one patient-independent or universal feature, such as, for example, universal cement wells 312 or other universal features. Such universal features can be used with any internal geometry 314, which can be machined into the generic casting 300 to accommodate the appropriate shape and/or size for a specific patient.

It will be appreciated from the above discussion that generic casting can greatly reduce inventory, machining costs and investment in mold tooling, while at the same time accommodating sizes and geometric features specific to a patient. Specifically, each implant type can be formed from a generic casting that can accommodate multiple sizes, such as four sizes, for
example. For implants that are available in eight sizes, generic casting can reduce inventory by a half, using two molds total for eight sizes. Further, additional reductions in inventory can be obtained by combining right and left side implants into a single generic casting, as discussed above in relation to FIG. 4.

The foregoing discussion discloses and describes merely exemplary arrangements of the present teachings. Furthermore, the mixing and matching of features, elements and/or functions between various embodiments is expressly contemplated herein, so that one of ordinary skill in the art would appreciate from this disclosure that features, elements and/or functions of one embodiment may be incorporated into another embodiment as appropriate, unless described otherwise above. Moreover, many modifications may be made to adapt a particular situation or material to the teachings of the invention without departing from the essential scope thereof. One skilled in the art will readily recognize from such discussion, and from the accompanying drawings and claims, that various changes, modifications and variations can be made therein without departing from the scope of the present invention as defined in the following claims.

## Claims

1. An orthopedic implant manufacturing method comprising:
preparing a preliminary pre-operative surgical plan for a specific patient (130) including a first option (170) for a patient-specific implant customized for the specific patient, a second option (180) for a semi-custom implant that has at least one patient-independent feature and at least one patient-specific feature, and a third option (190) for a non-custom implant available only in predetermined sizes;
communicating the plan to a surgeon of the patient (140);
receiving an orthopedic implant design recommendation of the surgeon (140), the implant design recommendation selecting one of first option (170), the second option (180) and the third option (190);
sending a request for manufacturing the selected implant to a manufacturing center;
manufacturing the selected implant based upon the request (250 or 240 or 200) receiving the implant;
and forwarding the implant for implantation (290).

2. The method of claim 1, further comprising receiving an alignment guide design recommendation from the surgeon (140), providing the alignment guide and implant to the surgeon in one package.

3. The method of claim 1, further comprising including a disposable instrument in the package.

4. The method of claim 1, wherein the semi-custom made implant is customizable to the patient from a generic casting of a specific implant component (240).

5. The method of claim 4, further comprising:
selecting the semi-custom implant (180); and
machining at least one geometric feature of the generic casting for a size and shape specific to the patient (210).

6. The method of claim 5, further comprising machining a height and a width of the implant specific to the patient (210).

7. The method of claim 5, further comprising casting a cement well in an internal surface of the implant (240).

## Patentansprüche

1. Verfahren zum Herstellen eines orthopädischen Implantats, umfassend:
Erstellen eines vorläufigen präoperativen Operationsplans für einen spezifischen Patienten (130), der eine erste Option (170) für ein patientenspezifisches Implantat, das an den spezifischen Patienten angepasst ist, eine zweite Option (180) für ein teilweise angepasstes Implantat, das zumindest ein patientenunabhängiges Merkmal und zumindest ein patientenspezifisches Merkmal aufweist, und eine dritte Option (190) für ein nicht angepasstes Implantat, das nur in vorbestimmten Größen verfügbar ist, beinhaltet;
Kommunizieren des Plans an einen Chirurgen des Patienten (140);
Erhalten einer Designempfehlung für das orthopädische Implantat von dem Chirurgen (140), wobei die Designempfehlung für das Implantat eine von der ersten Option (170), der zweiten Option (180) und der dritten Option (190) auswählt;
Senden einer Anforderung zur Herstellung des ausgewählten Implantats an ein Herstellungszentrum;
Herstellen des ausgewählten Implantats auf Grundlage der Anforderung (250 oder 240 oder 200); Erhalten des Implantats;
und Weitergeben des Implantats zur Implantation (290).

2. Verfahren nach Anspruch 1, ferner umfassend Erhalten einer Designempfehlung für eine Ausrichtungsführung von dem Chirurgen (140), Bereitstellen der Ausrichtungsführung und des Implantats an den Chirurgen in einem Paket.

3. Verfahren nach Anspruch 1, ferner umfassend Einschließen eines wegwerfbaren Instrumentes in das Paket.

4. Verfahren nach Anspruch 1, wobei das teilweise angepasste Implantat aus einem generischen Gussteil einer spezifischen Implantatskomponente (240) an den Patienten anpassbar ist.

5. Verfahren nach Anspruch 4, ferner umfassend:
Auswählen des teilweise angepassten Implantats (180); und Bearbeiten von zumindest einem geometrischen Merkmal des generischen Gussteils auf eine für den Patienten (210) spezifische Größe und Form.

6. Verfahren nach Anspruch 5, ferner umfassend Bearbeiten einer für den Patienten (210) spezifischen Höhe und Breite des Implantats.

7. Verfahren nach Anspruch 5, ferner umfassend Gießen einer Zementvertiefung in einer Innenfläche des Implantats (240).

## Revendications

1. Procédé de fabrication d'un implant orthopédique comprenant :
la préparation d'un plan chirurgical préopératoire préliminaire pour un patient spécifique (130) comprenant une première option (170) pour un implant spécifique au patient personnalisé pour le patient spécifique, une deuxième option (180) pour un implant semi-personnalisé qui présente au moins une caractéristique indépendante du patient et au moins une caractéristique spécifique au patient, et une troisième option (190) pour un implant non personnalisé disponible uniquement dans des tailles prédéfinies ;
la communication du plan à un chirurgien du patient (140) ;
la réception d'une recommandation de conception d'implant orthopédique du chirurgien (140), la recommandation de conception d'implant sélectionnant une option parmi la première option (170), la deuxième option (180) et la troisième option (190) ;
l'envoi d'une demande de fabrication de l'implant sélectionné à un centre de fabrication ;
la fabrication de l'implant sélectionné sur la base de la demande (250 ou 240 ou 200) ;
la réception de l'implant ; et
l'acheminement de l'implant en vue de l'implantation (290).

2. Procédé selon la revendication 1, comprenant en outre la réception d'une recommandation de conception de guide d'alignement en provenance du chirurgien (140), la fourniture du guide d'alignement et de l'implant au chirurgien en un seul paquet.

3. Procédé selon la revendication 1, comprenant en outre l'inclusion d'un instrument jetable dans le paquet.

4. Procédé selon la revendication 1, ledit implant fabriqué semi-personnalisé pouvant être personnalisé au patient à partir d'un moulage générique d'un composant d'implant spécifique (240).

5. Procédé selon la revendication 4, comprenant en outre :
la sélection de l'implant semi-personnalisé (180) ; et
l'usinage d'au moins une caractéristique géométrique du moulage générique pour obtenir une taille et une forme spécifiques au patient (210).

6. Procédé selon la revendication 5, comprenant en outre l'usinage d'une hauteur et d'une largeur de l'implant spécifique au patient (210).

7. Procédé selon la revendication 5, comprenant en outre le moulage d'un puits de ciment dans une surface interne de l'implant (240).
